# EUROPEAN PATENT APPLICATION

(11) **EP 2 065 069 A1**
(43) Date of publication of application: **03.06.2009**
(21) Application number: 07121923.2
(22) Date of filing: 29.11.2007
(51) Int. Cl.: A61M 25/00

(54) **A Catheter based Medical Device and Method for Delivery of a Therapeutic Agent**

(71) Applicant: National University of Ireland Galway, Galway (IE)
(72) Inventor: Sheridan, William Stephen, County Cavan (IE); Murphy, Bruce Philip, Galway (IE); McHugh, Peter, Galway (IE); Vard, John, Dublin (IE)
(74) Representative: Lucey, Michael

(57) **Abstract**

The invention provides medical device for delivery of a therapeutic agent comprising: a body capable of moving to a target site, for delivery of the therapeutic agent, in a forward direction and at least one needle mounted for movement with respect to said body along its central axis for selective movement away from said body. A moveable central core, comprising a wedge, is adapted to move, in the opposite direction to said body, to contact said needle to deploy the needle end away from said central axis to engage said target site for delivery of the therapeutic agent. The inventive device is designed primarily to deliver therapeutic agents/components locally to a diseased artery wall. One of the invention's main advantages is that it does not require a balloon and provides a novel method to advance and retract delivery needles to deliver therapeutic agents/ compounds locally.

## Description

### Field of the Invention

The present invention relates to a catheter based medical device for local delivery of therapeutic agents to a body cavity, for example an artery wall.

### Background to the Invention

It is generally known that several medical conditions can be treated more effectively by local administration of therapeutic agents. Recent studies of the biology of the arterial wall have clarified the nature of several localized pathologic changes in the intima. which could be treated effectively with local administration of variety of pharmacologic agents. Examples of these include the vulnerable plaque (an atherosclerotic lesion with high probability of rapid evolution into total occlusion, resulting in dependent tissue death), and the restenosis lesions (the fibro cellular proliferative response to the trauma caused by angioplasty interventions onto the vascular wall, leading to recurrent blockage of treated vessel).

Active biological processes in a localizable segment of the circulation can potentially be addressed directly to a diseased area by local administration of active therapeutic agents more effectively than by the systemic administration route. The process of deploying a balloon, either for angioplasty or deploying a stent, causes injury to the blood vessel wall. This approach is problematic as damage can lead to neo-intimal tissue formation which can lead to the re-narrowing of the blood vessel.

Peripheral arterial disease (PAD) in the leg can cause severe loss of blood flow to the foot and in some cases amputation of the foot may be required. Although PAD is very similar to coronary artery disease in its formation, the treatments for the disease differs greatly. In the coronary arteries drug eluting stents are implanted and have excellent clinical results; however the heart is an embedded organ and is well protected from any external mechanical loads, while PAD usually occurs near the surface and the diseased arteries receive no such protection from external forces. Stainless steel or cobalt chromium stents may not be used to treat PAD because if they come into contact with any external loads they will deform permanently and lead to a substantial blockage within the vessel. Delivery of therapeutic agents to the peripheral diseased vessel wall would be preferable to stenting as it provides a temporary mechanical solution (balloon angioplasty) and a permanent biological solution (possibly gene therapy), rather than a permanent mechanical solution (stenting), to a biological problem.

Arteriovenous (AV) fistulas are created by connecting an artery directly to a vein, they are often created as an access port for hemodialysis patients with end stage renal disease. The fistula is usually created in an arm or a leg. Stenosis of these AV fistulas is extremely common and requires surgery to unblock them, often with poor results. Percutaneous intervention is also used to unblock the stenosis, however they are usually highly fibrotic and difficult to treat. Angioplasty balloons with high burst pressures (over 20 atmospheres) are used and also cutting balloons, however, restenosis rates remain extremely high. There is therefore a need to provide a device or method to reduce restenosis rates.

Other catheter delivery systems use infusion techniques in order to deliver drugs and other therapeutics agents. Infusion techniques have been proven to be inferior to needle based delivery systems (Gonshior et al, American Heart Journal, Volume 130, Number 6, December 1995). These techniques can involve blocking arteries through the use of occluding balloons and exposing them to a solution of the agents, by the process of diffusion the agents pass into the vessel wall. Larger amounts of drugs may be needed compared to the required quantity. Current techniques of infusion or direct injection of therapeutic agents for local delivery have efficiencies of approximately 0.1% to 1% (Bailey et al, Current Interventional Cardiology Reports 2000, No. 2, p 349-357). Although this figure is extremely low it represents a 100-fold increase in comparison to systemic delivery. This clearly identifies the need for a more efficient, accurate, needle based low profile catheter system for the local administration of therapeutic agents.

Gene therapy and stem cell treatments are relatively new areas of research and early results are extremely promising. The constant development of new drugs, stem cells and gene therapy compounds requires substantial in-vivo testing, with many rounds of animal trials required before a new therapeutic can be considered safe for use in humans. There is a need to provide a device to effectively delivery these therapeutic agents to a target area. In addition there is a large amount of research being carried out in the use of stem cells to try and regenerate infracted tissue in the brain. There is already a trend in the development of devices that can reach areas of the brain and deliver therapeutic agents. This is seen by the products of companies, such as eV3, which use perfusion guide wires and guide catheters. Future treatment directions in cerebrovascular disease seem to be heading in the direction of locally delivering therapeutics using extremely low profile devices.

Even though drug eluting stents have reduced in-stent restenosis significantly a certain level of edge-stent restenosis still exists. *Alfonso et al* carried out a study on edge stent restenosis and determined the problem was recognised in a unique subgroup of patients (ACC Current Journal Review, Volume 14, Issue 2, February 2005, Page 47). This study concluded that the optimal treatment for such a condition is to carry out a repeat stent implantation as opposed to conventional balloon angioplasty. However a problem with these types of stents is that balloon initiated damage is caused to the artery wall upon balloon expansion. A needle based delivery catheter can eliminate this damage while injecting anti-proliferative drugs to the artery wall proximal and distal to a stent.

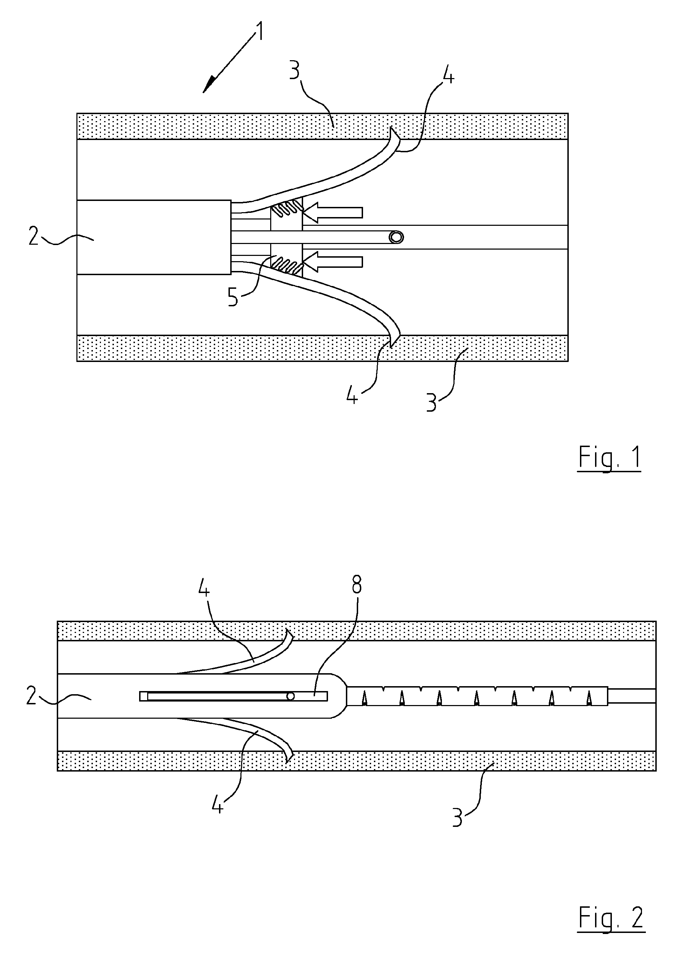

Needle based catheter devices for the local administration of therapeutic agents are known, for example as disclosed by US Patent No. 5,419,777. US Patent No. 6,997,903, Wijay et al*,* discloses a drug delivery device comprising a plurality of needles contained within an injection head. As shown in Figure 2 a force can be supplied by a cam, in the direction the device has travelled to reach the target site, to urge the needles outwards to a vessel wall for delivery of the therapeutic product to the target site. A problem with this device is that is difficult to control movement of the needles and also to retract the needles after delivery.

Heretofore, no satisfactory solution has been proposed to overcome the above mentioned problems with needle based local delivery of therapeutic agents.

### Summary of the Invention

According to the invention there is provided, as set out in the appended claims, a medical device for delivery of a therapeutic agent comprising: a body capable of moving to a target site, for delivery of the therapeutic agent, in a forward direction; at least one needle mounted for movement with respect to said body along its central axis for selective movement away from said body; a moveable central core comprising a wedge adapted to move, in the opposite direction to said body, to contact said needle to deploy the needle end away from said central axis to engage said target site for delivery of the therapeutic agent.

The device of the present invention is designed primarily to deliver therapeutic agents/components locally to the diseased artery wall. The invention's main advantage is that it does not require a balloon and has a novel method to advance and retract delivery needles to deliver compounds locally. Minimal vessel wall contact is made with this device due to the absence of a balloon and the contact that is made is extremely localized to the tip of the needle or needles, which reduces the overall damage to the artery wall and lowers the risk of restenosis. The absence of a balloon allows for a low crossing profile of the device, which is a huge advantage as the device can be delivered to various tortuous locations within the circulatory system. In addition the fact that the wedge moves backwards in relation to the forward movement of the catheter provides a greater level of control in deploying the needle

The low profile nature of the device would allow treatments to be carried out in locations where current interventional procedures display inferior results. Areas below the knee and in particular the foot would benefit from local drug delivery from the device of the present invention.

The invention solves the problem of local delivery of therapeutics agents to a body cavity, namely an artery or a vein. Such therapeutic agents include but are not limited to pharmaceuticals, stems cells and gene therapy products. The basic function of the device is to be manipulated to a desired site of disease within a blood vessel and accurately release therapeutic agents into the blood vessel wall. The device causes a minimal amount of damage to the vessel wall upon delivery to the target site and during the delivery of the therapeutic agents. The damage is limited by the localised contact with the vessel wall through the use of micro-needles. The device is extremely low in profile so that it can reach smaller diameter vessels that are currently difficult to treat with standard interventional procedures.

Ideally there is provided a retaining ring to securely hold the at least one needle along the central axis of said body in a retracted state. The retaining ring moves relative to the wedge movement to allow for deployment of the needle away from said central axis. It will be appreciated that the retaining ring can be any shape so long as its function is to hold the needles in a retracted or can force the deedless to retract from a deployed position. The distance between the wedge and the retaining ring determines the amount the needle is deployed. In one embodiment the wedge comprises means for engaging the central axis such that the distance between the wedge and the retaining ring can be selected when an external rotational force is applied to the central axis. A suitable means for engaging the central axis is to have a threaded portion on the central axis to communicate with a threaded portion of the retaining ring and/or the moveable core.

The needles are positioned in such a way that they can be deflected from the central axis of the device body toward a vessel wall. The wedge component of the central core functions to force the needles from a parallel position into a diseased vessel wall. The function of the retaining ring is to initially conceal the needles and retract them back into the device in a controlled manner. It will be appreciated that deployment of the needles is achieved through the displacement of the moveable central core, with the wedge and the retaining ring attached. The separation distance between the wedge and the retaining ring controls the deployment force and the depth of the needle penetration into the vessel wall. This minimises the needle length therefore providing a greater degree of flexibility.

Suitably, the needle is curved to aid in the deployment of the needle when contacted by said wedge. The needle can be provided with a second curve such that the needle end is positioned substantially perpendicular to said central axis when deployed.

In one embodiment the wedge is substantially in the shape of a truncated cone. Suitably, the wedge comprises a curved chamfer positioned on the outer surface of the wedge for engaging the needle to deploy said needle when said wedge is moved. In a further embodiment the wedge is in the form of a resilient deformable piece of material.

In another embodiment the device is adapted to deploy at least one needle in close proximity to the proximal end of an inserted cage, for example a self-expanding stent, and a second needle adapted to be deployed in close proximity to the distal end of the inserted cage.

In another embodiment there is provided a stabilising element positioned on the end of the needle to contact said target site to stabilise the needle such that a needle tip can penetrate the target site for delivery of the therapeutic agent at a desired location. The stabilising element maybe dimensioned as a frusto-conical element such that the largest diameter contacts the target site to stabilise the needle.

In another embodiment there is provided a second needle positioned in close proximity to the first needle, each needle in separate fluid communication, wherein the first needle delivers a first therapeutic agent and the second needle delivers a second therapeutic agent. Ideally the first needle penetrates the target area to deliver the first therapeutic agent at a first depth and the second needle penetrates the target area to deliver the second therapeutic agent at a second depth.

Suitably, the needle is made from nitinol or other memory alloy.

In another embodiment there is provided a moveable core comprising a plunger adapted to move under pressure by action of a pressurised fluid to control the movement of said wedge. The main advantage this embodiment is the reduction in size that can be achieved by utilizing a hydraulic controlled plunger to deploy the needles, thus reducing the overall profile of the device.

According to the present invention there is further provided a method for delivering a therapeutic agent treating one or more target areas of a vessel wall within a human or animal body, the method comprising the steps of: moving a catheter body to the target area, for delivery of the therapeutic agent, in a forward direction; providing at least one needle mounted for movement with respect to said catheter body along its central axis for selective movement away from said catheter body; and moving a moveable central core comprising a core body, for example a wedge, in the opposite direction to said catheter body to contact said needle, to deploy the needle end away from said central axis to engage said target site for delivery of the therapeutic agent. Suitably the method comprises the additional step of providing a retaining ring to securely hold the at least one needle along the central axis of said body in a retracted state.

### Brief Description of the Drawings

The invention will be more clearly understood from the following description of an embodiment thereof, given by way of example only, with reference to the accompanying drawings, in which:-
Figure 1 illustrates a first embodiment of the device of the present invention;
Figure 2 illustrates a further embodiment of the device of Figure 1
Figure 3 is cross sectional view of Figure 2 along its central axis in a retracted state;
Figure 4 is a cross sectional view of Figure 2 along its central axis in an expanded state;
Figure 5 illustrates a preferred shape of a needle for use in conjunction with the device of the present invention;
Figure 6 shows an example of a wedge and retaining ring shape of the present invention;
Figure 7 illustrates a different embodiment of the present invention;
Figure 8 is another embodiment of the present invention;
Figure 9 is a further embodiment of the present invention;
Figure 10 illustrates another embodiment of the present invention;
Figure 11 illustrates an alternative embodiment of the present invention;
Figure 12 illustrates yet another embodiment of the present invention;
Figure 13 illustrates an alternative embodiment of the present invention;
Figure 14 shows the embodiment of Figure 13 in use;
Figure 15 illustrates yet another embodiment of the present invention; and
Figure 16 shows the embodiment of Figure 15 in use.

### Detailed Description of the Drawings

Referring now to Figure 1 there is illustrated a first embodiment of the present invention, indicated by the reference numeral 1. A catheter body 2 is capable of moving to a target site 3, for example an artery wall. At least one needle 4 is mounted for movement with respect to the catheter body 2 along its central axis for selective movement away from the catheter body 2. A moveable central core 5 comprising a core body, for example a wedge, is adapted to move, in the opposite direction to the catheter body 2 to contact the needle to deploy the needle end away from the central axis to engage the target site for delivery of a therapeutic agent. The large arrows indicate the movement of the wedge in the opposite direction in which the catheter body has moved. The movement of the core body 5 then forces the needle radially out and upward, as shown by the small arrows, towards the artery wall 3. Further movement of the core body allows the end of the needle (needle tip) to penetrate the artery wall to the target site as required. The therapeutic agent can be delivered to the desired target area via the end of the needle 4.

Referring to Figure 2 illustrates a further embodiment of the invention and uses same reference numerals to identify common components with Figure 1. The distal tip of the catheter body 2 is modified to provide four slotted openings 8 to allow the release (and concealment) of four needles 4. The body 2 encapsulates the needles and moveable core mechanism 5. A component 6 is attached to the movable core of the catheter along with the wedge. The component can best be described as a retaining ring, its function is to allow the elastic needles to be deployed in a controlled manner and to collapse the needles back in to the catheter head after a substance has been delivered into the artery wall. Figure 2 displays the device with the needles deployed and penetrating a vessel wall.

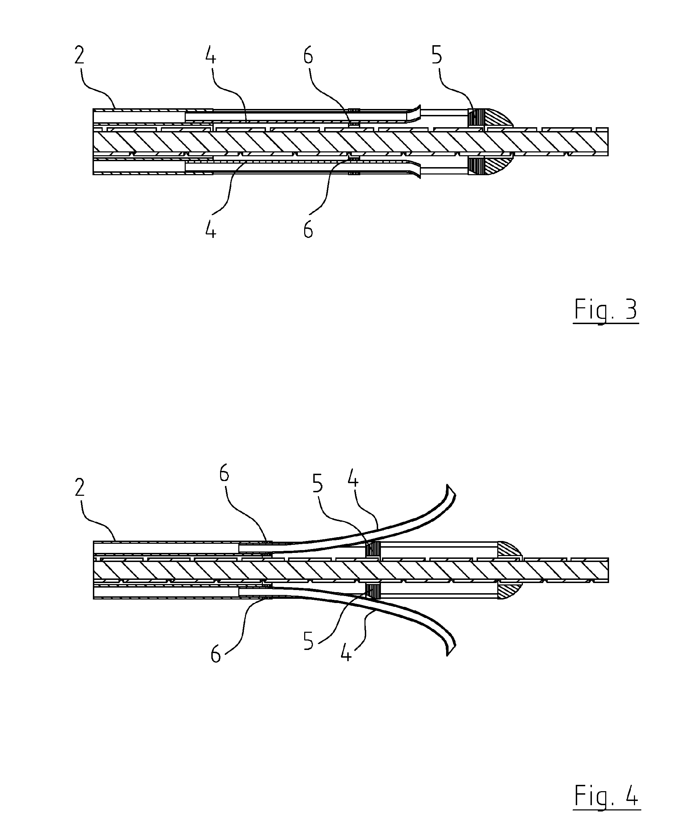

The structure of the device in both the deployed and collapsed states is shown in Figures 3 and 4. The device can be delivered upon a standard guidewire to the target location in the state shown in Figure 3, with the needles collapsed and concealed within the main catheter body 2. Once the device is confirmed to be in the desired position, for example through the use of radiopaque markers, the needles 4 may be deployed to deliver therapeutic agents to the target site. The moveable core is moved backwards by the operator which forces the wedge to contact the needles and causes their lateral deployment away from the central axis of the catheter, at the same time the retaining ring 6 moves backwards allowing the needles freedom to move outwards. The length of the needles and the distance between the wedge and retaining ring corresponds to the diameter of the vessel to be treated. Once the moveable core is moved backwards to its final location the needle ends 4 can penetrate the artery wall and can deliver therapeutic agents via the needle tip. The needles in a deployed state are shown in Figure 4.

Referring to Figure 3 the needles can then be collapsed in a controlled manner by moving the moveable core forwards which forces the retaining ring to push forward on the deployed needles and force them back into their collapsed position and concealed within the main catheter body 2 in a low profile state. Figure 3 shows the device with the needles retracted, an inward force is supplied by the retaining ring on the needles to keep them in their concealed position, while Figure 4 shows the device in the expanded configuration the retaining ring and the wedge move in the direction of the arrow and in this case the wedge exhibits an outward force on the needles forcing them into the artery wall.

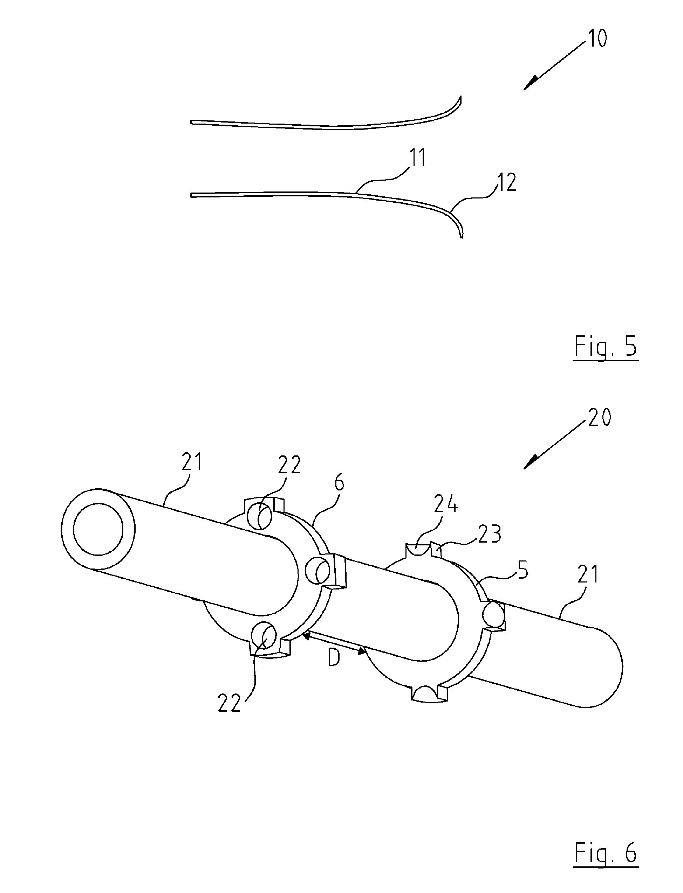

It will be appreciated needles for the device can be made from any suitable type of material known in the field of medical devices. The inventors have found that a particularly advantageous material is nitinol because it exhibits superelastic properties and has a relatively high modulus. The ability of nitinol to be "shape set" into any desired shape allowed for a curved distal tip of the needle to be created. The curvature permits the needle to be concealed within the catheter body and also for perpendicular penetration of the needle into the vessel wall. This allows for smoother movement of the wedge which in turn increases the positional control of the needle. Figure 5 illustrates the shape of a preferred embodiment of the needle, indicated generally by the reference numeral 10. The manufacturing process involved grinding the needles to sharp points once they had been set into the required shape.

Typically each needle co-operates with a therapeutic delivery lumen so that the therapeutic agent can be delivered to the needle in a conventional manner. It will be appreciated that the needle has a first curve 11 to aid in the deployment of the needle when contacted by the wedge to reduce friction. In addition the needle is provided with a second curve 12 such that the needle end is positioned substantially perpendicular to said central axis when deployed.

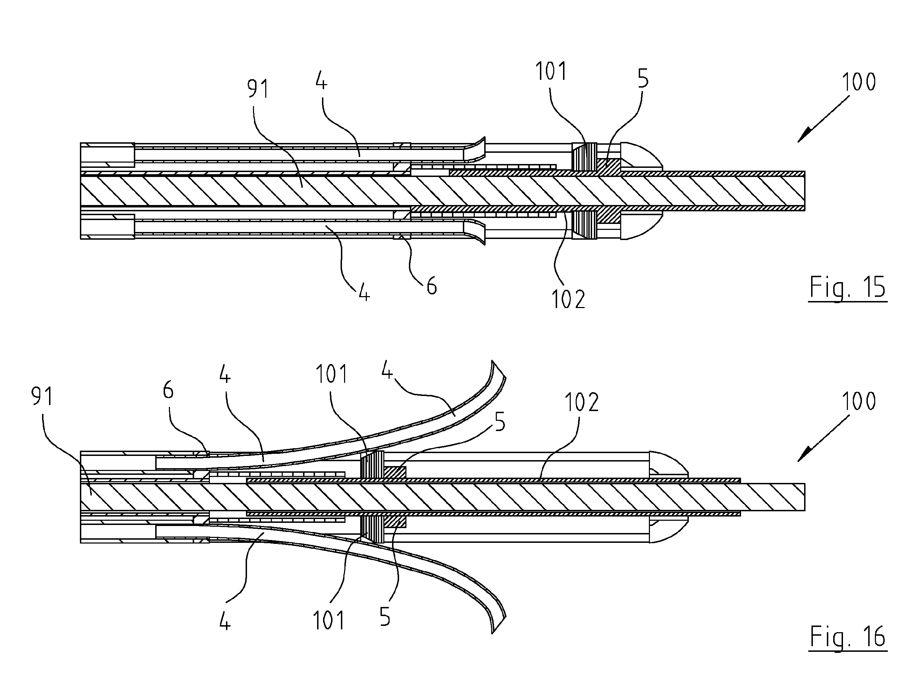

Referring to Figure 6 illustrates a central axis of the catheter body 21. The wedge 5 and the retaining ring 6 are shown in a typical embodiment, both of which can slide axially along the catheter body when a force is applied. The retaining ring 6 comprises a number of substantially circular openings 22 for receiving a needle in use. In a preferred embodiment there is provided four openings for receiving four separate needles. The wedge 5 may be designed with a chamfer 23 that can be curved 24 in the area the needle contacts the wedge in use. The curved surface 24 allows for smoother motion of the moveable core and efficient deployment of the needles in use. It will be appreciated that deployment of the needles can be achieved with any suitable shaped wedge. An important aspect of the invention is the separation distance between the wedge 5 and the retaining ring 6 indicated by the distance D in Figure 6. Advantageously the present invention allows the user to control the distance D depending on the application required. The distance D is proportional to the distance the needles are deployed. Thus, the force and the depth of the needle penetration into the vessel wall (target area) can be controlled by selection of the distance D. The medical device of the present invention can be selected for different sized applications. A suitable embodiment for controlling the distance D is discussed below with reference to Figures 15 and 16.

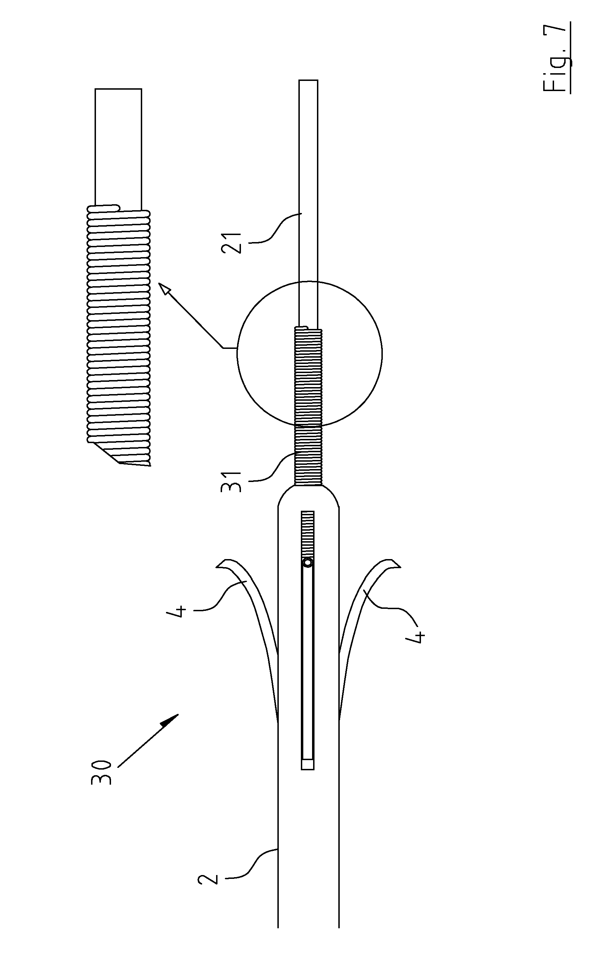

Referring to Figure 7 there is illustrated an alternative embodiment of the invention indicated by the reference numeral 30. In this embodiment a round wire coil 31 is used in communication with the moveable core and positioned towards the front of the moveable core. The coil 31 is resilient but offers the flexibility that is required at the distal tip to deliver the device to locations within tortuous locations/vessels. It will be appreciated that the coil 31 will able to transmit some or all of the required force from the moveable core/wedge over the full length of the catheter to fully deploy the needles without any deformation of the movable core, due to the resilient properties of the coil 31.

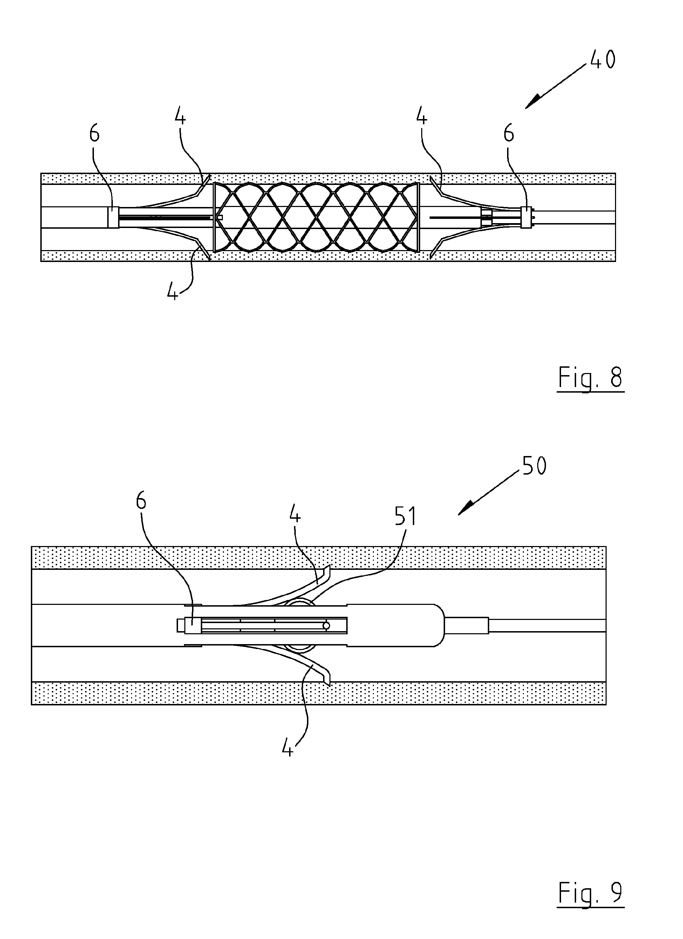

Figure 8 depicts the possible use of the device in conjunction with a self expanding stent or cage that is commonly used to treat peripheral arterial disease, indicated by the reference numeral 40. In this embodiment the moveable core 6 is pushed distally which forces the movement of the proximal needles 4 distally toward the proximal stent edge and up against a static wedge, furthermore a distal wedge moves towards the static distal needles forcing them outwards. The moveable core 6 is then pulled proximally by the operator which pulls the proximal needles from the vessel wall back to their original position and pulls a distal retaining ring over the distal needle to collapse them. The advantage of this embodiment is that when the stent has been deployed the therapeutics can easily be delivered to the edges of the stent to prevent edge stent restenosis.

Figure 9 illustrates a further embodiment to the invention and provides for the use of a self expanding nitinol cage instead of a wedge to deflect the needles, indicated by the reference numeral 50. The use of a nitinol cage 51 instead of the wedge can allow for further expansion of the needles as the cage would contact the needles at a higher point on the needles and can offer additional support to the needles. The nitinol cage 51 is constrained within a sheath (not shown) which can be pulled proximally in order to expand the cage 51. The retaining ring 6 is also attached to the sheath which allows for the collapse of the needles with its distal movement. The embodiment shown in Figure 9 provides additional support to the needles.

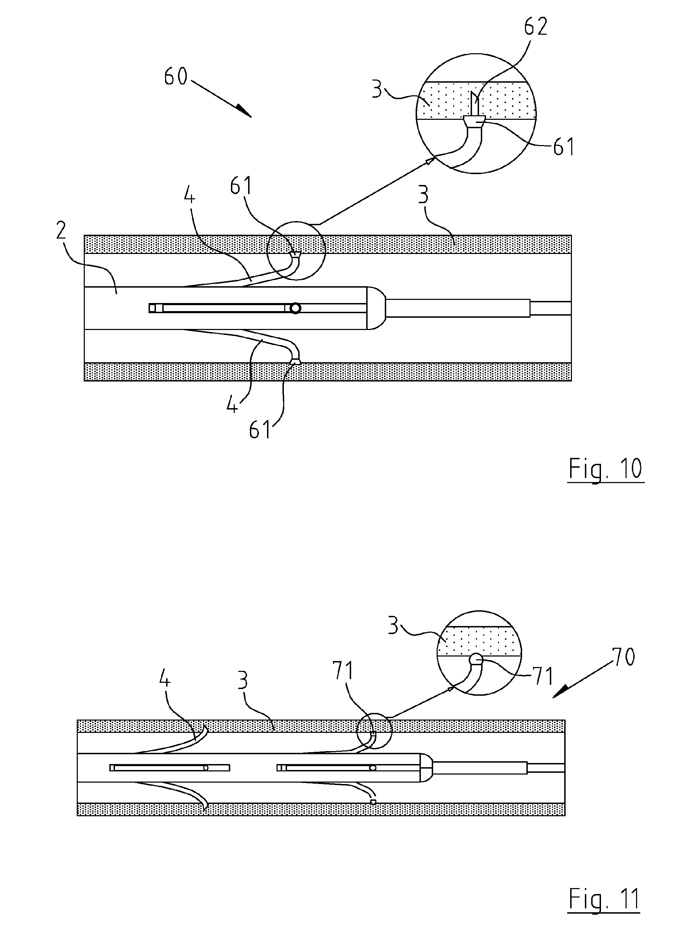

Referring to Figure 10 a further embodiment to the invention, indicated by the reference numeral 60 involves the use of a funnel shaped needle delivery apparatus to provide stability for the needle 4 before delivery of the therapeutic agent. The nitinol needle's tip is replaced with a funnel shaped tip 61 that does not penetrate the vessel wall 3. The funnel tip 61 contacts the wall of the vessel with minimal force such as to reduce the amount of damage due to contact with the endothelium. Once the funnel element 61 is deployed the device is stabilised and allows for the distal movement of a flexible nitinol needle tip 62 within the funnel element 61 to penetrate the vessel wall. These needle tips 62 can then be deployed as far as required into the vessel wall by the operator, depending on what therapeutic agents are being delivered. It will be appreciated that the any shaped stabilising element can be used so long as the device is stabilised. The advantage of this embodiment lies in the stability of the device and the localised minimal vessel wall contact.

Referring to Figure 11 illustrates a further embodiment of the invention involves the use of a stabilising device which aligns the device onto a central axis in order to minimise vessel damage from the needles during deployment, as indicated by the reference numeral 70. This embodiment may be required when using the device in areas of high blood pressure where the blood flow may disrupt the mechanism of needle deployment and force the device off centre. The stabilizing element 71 is simply a second wedge and retaining ring system, attached to the moveable core, which deflect nitinol toward the vessel wall, however, in this case the nitinol is not a sharpened needle and does not penetrate the vessel wall. The stabilising elements 71 contain a smooth spherical tip to anchor against the vessel wall providing support for the overall device but cause as little damage to the endothelium as possible. The distance between this second wedge and retaining ring is selected so that the stabilizing elements are deployed first and come into contact with the vessel prior to the needles. This forces the device onto the central axis of the vessel and allows for a uniform symmetrical expansion of the needles, with minimal risk of vessel damage.

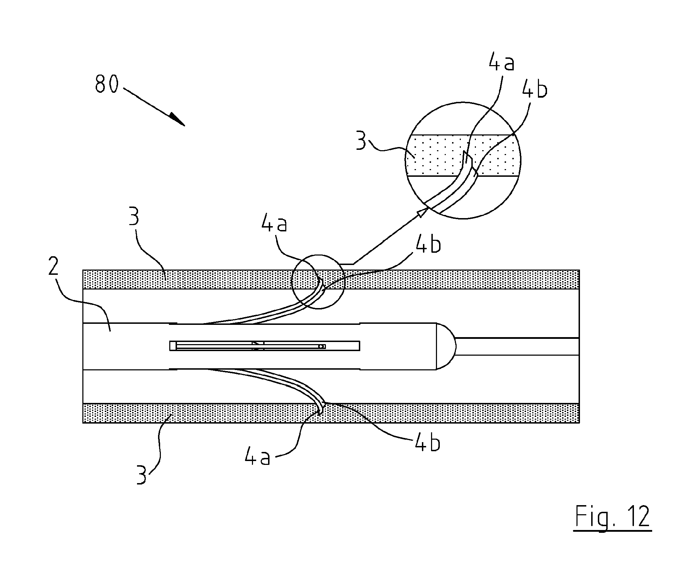

Figure 12 shows another embodiment to the invention which involves the use of a dual needle delivery system indicated by the reference numeral 80. The principal of this embodiment is that there is provided a first needle 4a and a second needle 4b. The second needle 4b is positioned in close proximity to the first needle 4a, each needle in separate fluid communication, wherein the first needle delivers a first therapeutic agent and the second needle delivers a second therapeutic agent. It will be appreciated that each needle 4a, 4b contains two lumens that are in separate fluid communication to different reservoirs of therapeutic agents. One lumen of the needle penetrates deep in the vessel wall in order to deliver agents to heal disease or present restenosis. The second lumen of the needle penetrates just inside the vessel wall and delivers a therapeutic agent that can heal the damage to the outer endothelial layer caused by the needle penetrating the vessel wall. As clearly illustrated in Figure 12, the first needle penetrates the target area to deliver the first therapeutic agent at a first depth and the second needle penetrates the target area to deliver the second therapeutic agent at a second depth. The various depths can be selected depending on the application required.

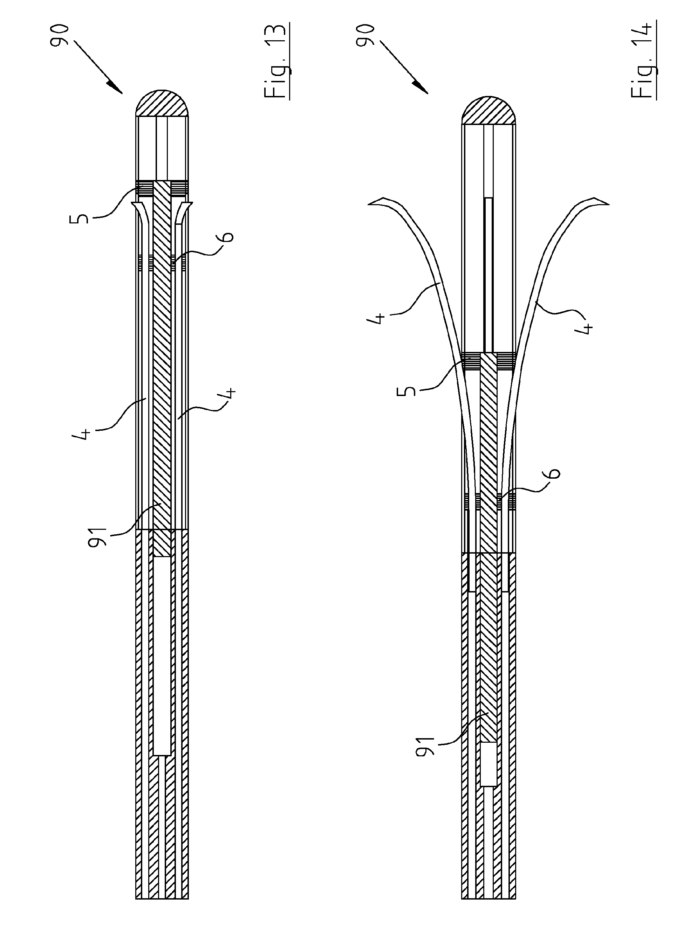

A further embodiment to the device is described below with reference to Figures 13 and 14, indicated by the reference numeral 90. Once more this embodiment makes use of a wedge 5 and a retaining ring 6 to deploy and collapse needles 4 to inject therapeutic compounds into a vessel wall. The moveable core described in the previous embodiments is in the form of a plunger 91 that is actuated by a compressed fluid, controlled by the operator. In the undeployed state shown in Figure 13 the plunger 91 is pressurised to maintain it at the distal end of the device, allowing the retaining ring 6 to constrain and conceal the needles. Once the fluid pressure is released the plunger 91 is pulled proximally which forces the wedge 5 to deflect the needles toward the vessel wall and allow delivery of a desired therapeutic. The device functions under the same premise as the main embodiment described in above but instead of the operator manually actuating the moveable core over the length of the device, the fluid pressure controls the plunger 91 and hence the deployment of the needles.

The main advantage of the embodiment shown in Figures 13 and 14 is the reduction in size that can be achieved by utilizing a hydraulic controlled plunger 91 to deploy the needles. The profile of the main catheter shaft can be produced to the same size, for example as a standard 0.014 inch guide wire and therefore, will allow any standard device, such as balloon catheters or balloon mounted stents to be placed over it. The device could also be delivered over a guide wire in a "rapid exchange" manner, whereby the rapid exchange entry port would be distal to the needles. The lack of a moveable core over the full length of the catheter also greatly increases the overall flexibility of the device and allows for ease of delivery to various tortuous locations within the vasculature.

Referring to Figures 15 and 16 there is illustrated an embodiment to allow a user to control the distance D for deploying the needles indicated by the reference numeral 100. The distance D is proportional to the distance the needles are deployed. Thus, the force and the depth of the needle penetration into the vessel wall (target area) can be controlled by selection of the distance D. This is achieved by providing a mechanism 101 in the device that allows the user to alter this distance "D" such that the mechanism provides a means for engaging the moveable core with the central axis of the device. An example of the mechanism 101 is to include a threaded portion in communication with the moveable core 5. The advantage being that the user can choose the desired amount of needle expansion for the corresponding vessel that he/she is working on at the time.

The device shown in Figures 15 and 16 still functions basically in the exact manner as disclosed in the previous embodiments above. The moveable core 5 is moved backwards which forces the wedge to deflect/expand the needles 4. The ring 6 collapses the needles with the forward movement of the moveable core 5. The difference is the moveable core now contains a distal tip section comprising a threaded portion 102 that can be connected through a self-locking thread to the mechanism 101. The distance D can be set by rotating of the distal tip to cause the moveable core 5 to be threaded backwards or forwards along the threaded portion 102 depending on which way the tip is rotated. Hence the distance between the moveable core 5 and the ring 6 can be controlled. The distance "D" that the needles are displaced radially is increased or decreased depending on the distance between the core and the retaining ring. It will be appreciated that the core is not directly attached to the distal tip but can be prevented from moving by a blocker on either side of it (the blockers are attached to the distal tip) and also by the slotted openings in the catheter to allow the core to be threaded forwards or backwards when the distal tip is rotated. In essence the embodiment shown in Figures 15 and 16 creates a "one size fits all" device depending on the application required.

The words "comprises/comprising" and the words "having/including" when used herein with reference to the present invention are used to specify the presence of stated features, integers, steps or components but does not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The invention is not limited to the embodiments hereinbefore described but may be varied in both construction and detail.

## Claims

1. A medical device for delivery of a therapeutic agent comprising:
a catheter body capable of moving to a target site, for delivery of the therapeutic agent, in a forward direction;
at least one needle mounted for movement with respect to said catheter body along its central axis for selective movement away from said catheter body; and
a moveable central core comprising a core body, for example a wedge, adapted to move in the opposite direction to said catheter body to contact said needle, to deploy the needle end away from said central axis to engage said target site for delivery of the therapeutic agent.

2. The device of claim 1 comprising a retaining ring to securely hold the at least one needle along the central axis of said body in a retracted state.

3. The device of claim 2 wherein the retaining ring moves relative to the core body movement to allow for deployment of the needle away from said central axis.

4. The device of any preceding claim wherein the distance between the wedge and the retaining ring can be selected to determine the amount the needle is deployed from said central axis.

5. The device of claim 4 wherein the wedge comprises means for engaging the central axis such that the distance between the wedge and the retaining ring can be selected when an external rotational force is applied to the central axis.

6. The device of any preceding claim wherein the needle is curved to aid in the deployment of the needle when contacted by said wedge.

7. The device of claim 6 wherein the needle is provided with a second curve such that the needle end is positioned substantially perpendicular to said central axis when deployed.

8. The device of any preceding claim wherein the wedge is substantially in the shape of a truncated cone.

9. The device of any preceding claim wherein the wedge comprises a curved chamfer positioned on the outer surface of the wedge for engaging the needle to deploy said needle when said wedge is moved.

10. The device of any preceding claim wherein the wedge comprises a round wire coil.

11. The device of any preceding claim wherein the wedge is in the form of a resilient deformable piece of material.

12. The device of any preceding claim adapted to deploy at least one needle in close proximity to the proximal end of an inserted cage, for example a self-expanding stent, and a second needle adapted to be deployed in close proximity to the distal end of the inserted cage.

13. The device of any preceding claim comprising a stabilising element positioned on the end of the needle to contact said target site to stabilise the needle such that a needle tip can penetrate the target site for delivery of the therapeutic agent at a desired location.

14. The device of claim 13 wherein the stabilising element is dimensioned as a frusto-conical element such that the largest diameter contacts the target site to stabilise the needle.

15. The device of any preceding claim comprising a second needle positioned in close proximity to the first needle, each needle in separate fluid communication, wherein the first needle delivers a first therapeutic agent and the second needle delivers a second therapeutic agent.

16. The device of claim 15 wherein the first needle penetrates the target area to deliver the first therapeutic agent at a first depth and the second needle penetrates the target area to deliver the second therapeutic agent at a second depth.

17. The device of any preceding claim wherein the needle is a nitinol needle.

18. The device of any preceding claim wherein the moveable core comprises a plunger adapted to move under pressure by action of a pressurised fluid to control the movement of said wedge.
